# EUROPÄISCHE PATENTSCHRIFT

(11) **EP 4 359 047 B1**
(45) Veröffentlichungstag und Bekanntmachung des Hinweises auf die Patenterteilung: **18.03.2026**
(21) Anmeldenummer: 22735112.9
(22) Anmeldetag: 15.06.2022
(51) Int. Cl.: A61M 11/04, A61M 15/06

(54) **VERDAMPFERKARTUSCHE SOWIE INHALATOR MIT EINER SOLCHEN VERDAMPFERKARTUSCHE**
VAPORIZER CARTRIDGE AND INHALER COMPRISING SUCH A VAPORIZER CARTRIDGE
CARTOUCHE DE VAPORISATEUR ET INHALATEUR COMPRENANT UNE TELLE CARTOUCHE DE VAPORISATEUR

(30) Priorität: 22.06.2021 DE 102021116108
(43) Veröffentlichungstag der Anmeldung: 01.05.2024
(73) Patentinhaber: Körber Technologies GmbH, 21033 Hamburg (DE)
(72) Erfinder: MÜLLER, Thomas, 20259 Hamburg (DE)
(74) Vertreter: Stork Bamberger Patentanwälte PartmbB
(86) Internationale Anmeldenummer: PCT/EP2022/066308
(87) Internationale Veröffentlichungsnummer: WO 2022/268603

(56) Entgegenhaltungen:
- WO-A1-2015/161553
- WO-A1-2021/062781
- US-A1- 2014 286 630
- US-A1- 2017 273 360
- US-A1- 2017 281 883
- US-A1- 2018 116 289
- US-A1- 2019 014 826
- US-A1- 2020 113 242
- US-A1- 2020 196 669
- US-A1- 2021 127 744

## Beschreibung

Die Erfindung betrifft eine Verdampferkartusche als Bestandteil eines Inhalators, umfassend einen Hohlkörper mit einem durchgängigen, einen Strömungskanal bildenden, barrierefreien Schlot sowie einen Vorratstank zum Bevorraten von Flüssigkeit, wobei der Vorratstank mindestens eine Zugangsöffnung zum Schlot aufweist und im Bereich jeder Zugangsöffnung eine sich über die gesamte Zugangsöffnung erstreckende Verdampfereinheit angeordnet ist, die ein Dochtorgan und ein Heizorgan zum Bilden von Aerosolen aus der aus dem Vorratstank zugeführten Flüssigkeit aufweist, wobei die Verdampfereinheit dazu flüssigkeitspermeabel ausgebildet ist, derart, dass Flüssigkeit mindestens initial kapillar aus dem Vorratstank durch die Verdampfereinheit in Richtung des Schlotes und als Aerosol innerhalb des Schlotes in Richtung eines Mundstücks förderbar ist, wobei im Bereich des Schlotes mindestens abschnittsweise mindestens ein Absorptionselement angeordnet ist, das zum Aufnehmen von innerhalb des Schlotes durch Kondensation des Aerosols gebildeten Flüssigkeitstropfen ausgebildet und eingerichtet ist, wobei das Absorptionselement durch den Schlot selbst gebildet ist.

Des Weiteren betrifft die Erfindung einen Inhalator, ausgebildet und eingerichtet zum Inhalieren von mit Wirkstoffen angereichertem Aerosol, umfassend einen mindestens eine elektronische Steuereinheit und eine Energiequelle umfassenden Kartuschenträger sowie eine Verdampferkartusche.

Solche Verdampferkartuschen und Inhalatoren kommen in der Genussmittelindustrie, hier insbesondere im Zusammenhang mit einer elektronischen Zigarette, der so genannten E-Zigarette, sowie im medizinischen Bereich zum Einsatz, um fluide Genussmittel und/oder fluide medizinische Produkte inhalieren zu können. Verdampferkartuschen und Inhalatoren sind aus nachfolgenden Offenbarungen bereits bekannt: US 2017/281883 A1, US 2018/116289 A1, US 2020/113242 A1, US 2019/014826 A1, US 2021/127744 A1, US 2017/273360 A1, WO 2021/062781 A1, US 2020/196669 A1, WO 2015/161553 A1 und US 2014/286630 A1.

Im Zusammenhang mit dieser Erfindung umfasst der Begriff Aerosol auch Dampf bzw. Aerosol-Dampf-Gemisch. Beim Konsumieren saugt üblicherweise eine Person an einem Mundstück des Inhalators, wodurch in dem Strömungskanal ein Saugdruck entsteht, der einen Luftstrom durch den Strömungskanal erzeugt. Der Luftstrom kann aber auch maschinell z.B. durch eine Pumpe erzeugt werden. In dem Strömungskanal wird dem Luftstrom eine von der Verdampfereinheit erzeugte und bereitgestellte verdampfte Flüssigkeit als Gasphase zugegeben, um der konsumierenden Person ein Aerosol oder ein Aerosol-Dampf-Gemisch zu verabreichen. Die Flüssigkeit ist an der oder in der Verdampferkartusche bevorratet. Als Flüssigkeit kommen unterschiedliche Mischungen mit verschiedenen Bestandteilen gleicher oder unterschiedlicher Dampfdichten zum Einsatz. Eine typische Mischung für den Einsatz in einer E-Zigarette weist z.B. Bestandteile von Glycerin und Propylenglycol auf, ggf. angereichert um Nikotin und/oder nahezu beliebige Geschmacksstoffe. Für den Einsatz im medizinischen oder therapeutischen Bereich, z.B. zur Inhalation von Asthma-Präparaten, kann die Mischung entsprechend medizinische Bestandteile und Wirkstoffe aufweisen.

Die einzelnen Bestandteile der Verdampferkartusche, nämlich der Hohlkörper, der Vorratstank und die Verdampfereinheit, können in einem gemeinsamen Bauteil zusammengefasst sein, wobei dieses Bauteil dann ein Einwegartikel ist, der für eine endliche Anzahl von Inhalationszügen durch eine konsumierende Person ausgelegt ist und zusammen mit einem Kartuschenträger als wiederverwendbarem Mehrwegartikel, der mindestens eine elektronische Steuereinheit und eine Energiequelle umfasst, einen Inhalator bildet. Die Verdampferkartusche kann jedoch auch erst durch das Zusammenfügen mehrerer Bauteile gebildet sein, wobei einzelne Bauteile, nämlich insbesondere der Hohlkörper und die Verdampfereinheit, in dem Kartuschenträger als Mehrwegartikel angeordnet sind, und der Vorratstank als separates Bauteil den Einwegartikel bildet. Letztlich lässt sich der Inhalator durch Austausch des Einwegartikels, der üblicherweise die Flüssigkeit beinhaltet, variabel einsetzen.

Entsprechend sind der Einwegartikel und der Mehrwegartikel lösbar miteinander verbunden. Der Kartuschenträger als Mehrwegartikel umfasst üblicherweise mindestens eine elektronische Steuereinheit und eine Energiequelle. Die Energiequelle kann z.B. eine elektrochemische Einwegbatterie oder ein wiederaufladbarer elektrochemischer Akku, z.B. ein Li-lonen-Akku sein, mittels dem das Heizorgan über die elektrischen Kontakte der Verdampfereinheit mit Energie versorgt wird. Die elektronische und/oder elektrische Steuereinheit dient zum Steuern der Verdampfereinheit innerhalb der Verdampferkartusche. Der Kartuschenträger kann aber auch Bestandteile der Verdampferkartusche umfassen. Der Einwegartikel kann als Ansteckteil an den Mehrwegartikel ansteckbar oder als Einsetzteil in den Mehrwegartikel einsetzbar ausgebildet sein. Anstelle einer Steckverbindung sind auch Schraubverbindungen oder andere Schnellverbindungen einsetzbar. Mit der Verbindung von Einwegartikel und Mehrwegartikel wird eine mechanische und elektrische Kopplung zur Bildung eines funktionsbereiten Inhalators hergestellt.

Die zentrale und letztlich die Nutzung (z.B. als E-Zigarette oder als medizinischer Inhalator) bestimmende Komponente ist der mit einer Flüssigkeit (im Folgenden auch als Fluid bezeichnet) befüllte Vorratstank als Bestandteil der Verdampferkartusche. Mittels der flüssigkeitspermeablen Verdampfereinheit wird das Fluid aus dem Vorratstank aufgrund zumindest initial kapillarer Förderung durch das Dochtorgan und das Heizorgan geleitet. Die von der Energiequelle erzeugte Spannung, die an dem Heizorgan angelegt wird, führt zu einem Stromfluss im Heizorgan. Aufgrund des Heizwiderstandes, vorzugsweise des Ohm'schen Widerstands des Heizorgans, führt der Stromfluss zu einer Erhitzung des Heizorganes und letztlich zu einer Verdampfung des in der Verdampfereinheit befindlichen bzw. durch die Verdampfereinheit geführten Fluids. Das auf diese Weise erzeugte Gas bzw. der Dampf, das Aerosol und/oder das Aerosol-Dampfgemisch entweicht aus der Verdampfereinheit in Richtung des Strömungskanals. In dem durch den Schlot gebildeten Strömungskanal wird die Bildung von Aerosolen bzw. Aerosol-Dampfgemisch durch Mischung mit der Luftströmung innerhalb des Strömungskanals gezielt unterstützt. Das Fluid hat damit einen vorgegebenen Weg mit einer vorgegebenen Strömungsrichtung, nämlich als Fluid durch das Dochtorgan an das und durch das Heizorgan und als Gas/Dampf/Nebel und/oder Aerosol aus dem Heizorgan in den Strömungskanal bzw. Schlot. In dem Strömungskanal bzw. Schlot wird das verdampfte Fluid durch den Luftstrom mitgerissen, wenn der Strömungskanal bzw. Schlot mit einem Druck/Unterdruck beaufschlagt wird, indem z.B. eine konsumierende Person über das Mundstück an dem Strömungskanal saugt oder eine Pumpe einen Luftstrom durch den Strömungskanal bzw. Schlot pustet.

Damit das Fluid aus dem Vorratstank nicht direkt in den Strömungskanal bzw. Schlot fließt, deckt die Verdampfereinheit den Zugang vom Vorratstank zum Strömungskanal bzw. Schlot vollständig ab. Vollständig abgedeckt bedeutet in diesem Zusammenhang, dass die Flüssigkeit zwingend durch die Verdampfereinheit geführt ist, so dass das Fluid nicht direkt vom Vorratstank in den Strömungskanal bzw. Schlot gelangen kann, sondern den "Umweg" über das Dochtorgan und das Heizorgan nehmen muss. Das Dochtorgan dient zum einen zum Zwischenspeichern von Fluid, um insbesondere bei nahezu entleertem Vorratstank noch ausreichend Fluid für wenige Züge am Inhalator zur Verfügung zu stellen. Das Dochtorgan dient zum anderen insbesondere zum Transport des Fluids vom Vorratstank in Richtung des Strömungskanals bzw. Schlotes und wirkt gleichzeitig als eine Art Rückschlagschutz, um den Rücklauf von Fluid und/oder Dampf in Richtung des Vorratstanks zu unterbinden.

Bei der gattungsgemäßen Verdampferkartusche ist der Schlot durchgängig und barrierefrei ausgebildet. Der Schlot bildet dabei einen durchgängigen Strömungskanal. Das bedeutet, dass die Aerosole bzw. Aerosol-Dampf-Gemische innerhalb des Schlotes bzw. Strömungskanals frei in Richtung des Mundstücks strömen können. Der vom Schlot umschlossene Hohlraum ist frei von Hindernissen, so dass eine direkte und freie Kanalverbindung aus dem Bereich der Verdampfereinheit bis zum Mundstück existiert. Die dem Schlot zugeführten bzw. in diesem gebildeten Aerosole bzw. Aerosol-Dampf-Gemische strömen ungehindert in Richtung Mundstück. Barrierefrei im Sinne der Erfindung bedeutet im Idealfall, dass der Strömungskanal im Querschnitt konstant ausgebildet ist und eine glatte Oberfläche aufweist, um eine laminare Strömung zu ermöglichen. Barrierefrei schließt jedoch ausdrücklich auch Ausführungsformen mit ein, bei denen der Querschnitt des Strömungskanal ausgehend von der Verdampfereinheit bis zum Mundstück veränderlich bzw. nicht konstant ist und/oder im Strömungskanal Vorsprünge, Kanten, Erhebungen oder dergleichen vorgesehen sind, die sich jedoch nur teilweise über den Querschnitt des Strömungskanals erstrecken und von den Aerosolen bzw. Aerosol-Dampfgemischen umströmt werden können. Mit anderen Worten ermöglicht der barrierefreie Strömungskanal das Strömen der Aerosole bzw. Aerosol-Dampfgemische in Richtung Mundstück, ohne dass diese durch im Strömungskanal befindliche Komponenten oder dergleichen hindurchströmen müssen.

Durch die Abkühlung der Aerosole bzw. Aerosol-Dampfgemische auf ihrem Weg zum Mundstück u.a. an der Innenwand des den Strömungskanal umgebenden Schlotes entstehen durch Kondensation Flüssigkeitstropfen. Anders ausgedrückt werden die Aerosole bzw. Aerosol-Dampfgemische beim Transport von der Verdampfereinheit zum Mundstück zumindest teilweise wieder in Flüssigkeit umgewandelt. Durch den bestehenden Luftzug innerhalb des Schlotes bzw. Strömungskanals besteht die Gefahr, dass die flüssigen Tropfen in den Mund der nutzenden Person gelangen. Die Flüssigkeitstropfen beeinträchtigen zum einen das Genuss- und Geschmackserlebnis insbesondere im Zusammenhang mit dem Einsatz als E-Zigarette. Im Weiteren kann die direkte Aufnahme z.B. von Nikotin aus der Flüssigkeit über die Mundschleimhäute zu unerwünschten Effekten, z.B. Übelkeit führen. Zum anderen können die flüssigen Tropfen das Einnahmeerlebnis insbesondere im Zusammenhang mit einem medizinischen Inhalator beeinträchtigen.

Der Erfindung liegt somit die Aufgabe zugrunde, eine kompakte Verdampferkartusche der gattungsgemäßen Art zu schaffen, die das Austreten von Flüssigkeit aus dem Mundstück wirksam verhindert. Die Aufgabe besteht weiterhin darin, einen entsprechenden Inhalator vorzuschlagen.

Diese Aufgabe wird durch eine Verdampferkartusche der eingangs genannten Art dadurch gelöst, dass der Schlot zur Bildung eines Innenschlotes und eines Außenschlotes mindestens abschnittsweise doppelwandig ausgebildet ist, wobei der Außenschlot an den Vorratstank grenzt und entsprechend dicht ausgebildet ist, während der Innenschlot zur Herstellung einer Verbindung aus dem Strömungskanal in den Außenschlot mindestens abschnittsweise perforiert ausgebildet ist, wobei eine Wandung des Innenschlotes und eine Wandung des Außenschlotes zur Bildung eines Zwischenraums beabstandet zueinander angeordnet sind. Mit dem oder jedem Absorptionselement können sich bildende Fluidtröpfchen noch innerhalb des Schlotes aufgenommen werden, so dass diese gar nicht erst das Mundstück und erst recht nicht den Mund der inhalierenden Person erreichen. Mit anderen Worten können sich ggf. bildende Fluidtröpfchen noch vor dem Erreichen des Endes des Schlotes zurückgehalten werden, wodurch sich das Genuss- und Geschmackserlebnis bzw. das Einnahmeerlebnis signifikant verbessert.

Bevorzugt ist das oder jedes Absorptionselement entlang des sich von der Verdampfereinheit bis zum freien Ende des Mundstückes erstreckenden Abschnitts des Schlotes in Strömungsrichtung des Aerosols hinter der Verdampfereinheit angeordnet. Damit ist das oder jedes Absorptionselement präzise dort angeordnet, wo die Gefahr der potentiellen Tröpfchenbildung am größten ist, so dass ein wirkungsvoller Schutz gegen das unerwünschte Einnehmen/Einsaugen von Flüssigkeit geschaffen ist.

Erfindungsgemäß wird das Absorptionselement durch den Schlot selbst gebildet, der zur Bildung eines Innenschlotes und eines Außenschlotes mindestens abschnittsweise doppelwandig ausgebildet ist. Zum einen ist damit eine besonders kompakte Ausführung einer ein Absorptionselement aufweisenden Verdampferkartusche gebildet. Zum anderen wird die Gefahr der Tröpfchenbildung, also insbesondere das Kondensieren der Aerosole bzw. der Aerosol-Dampfgemische an der Innenwand des Schlotes, durch die Doppelwandigkeit reduziert, da das Aerosol bzw. Aerosol-Gas-Gemisch von der im Vorratstank bevorrateten Flüssigkeit quasi entkoppelt ist. Die Doppelwandigkeit wirkt quasi als Isolierung zum Vorratstank. Die Kondensatbildung an der Innenwand ist damit zumindest erschwert. Die Doppelwandigkeit kann sich über einen Teil der Strecke von der Verdampfereinheit bis zum Mundstück erstrecken. Optional kann auch die gesamte Strecke doppelwandig ausgebildet sein.

Erfindungsgemäß grenzt der Außenschlot an den Vorratstank und ist entsprechend dicht ausgebildet, während der Innenschlot zur Herstellung einer Verbindung aus dem Strömungskanal in den Außenschlot mindestens abschnittsweise perforiert ausgebildet ist. Mit der dichten Ausbildung des Außenschlotes gegenüber dem Vorratstank, der Außenschlot bildet bevorzugt eine Wand des Vorratstankes, ist eine flüssigkeitsdichte Abgrenzung beschrieben, derart, dass Flüssigkeit zuverlässig daran gehindert wird, in Richtung des Strömungskanals zu dringen, mit Ausnahme des Bereichs der Verdampfereinheit. Die mindestens abschnittsweise Perforierung, beispielsweise durch feinste Poren oder Bohrungen oder dergleichen in der Wandung des Innenschlotes, gewährleistet bzw. schafft eine Kapillarwirkung, derart, dass die sich an der Innenwand des Innenschlotes insbesondere durch Kondensation bildenden Tröpfchen, und auch jede andere Art von Flüssigkeit, durch die Öffnungen in Richtung des Außenschlotes wandern, also insbesondere aus dem Innenschlot abgeführt werden. Die Perforation oder jede andere Strömungsverbindung aus dem den Strömungskanal umgebenden Innenschlot in den Außenschlot kann sich abschnittsweise oder vollständig über den Schlot erstrecken. Die Perforation kann regelmäßig oder unregelmäßig ausgebildet sein, und beispielsweise in der Nähe der Verdampfereinheit ausgeprägter ausgebildet sein als im Bereich des Mundstückes oder umgekehrt.

Erfindungsgemäß sind die Wandung des Innenschlotes und die Wandung des Außenschlotes zur Bildung eines Zwischenraums beabstandet zueinander angeordnet. Damit ist ein Sammelraum für die aus dem Inneschlot in den Außenschlot geleitete Flüssigkeit geschaffen. Die dem Innenschlot entzogene Flüssigkeit kann so in dem Sammelraum gespeichert und ggf. über andere Kanäle abgeführt werden.

Eine besonders vorteilhafte Weiterbildung ist dadurch gekennzeichnet, dass im Zwischenraum ein zusätzliches Absorptionsmittel, z.B. in Form eines Vliesmaterials, angeordnet ist. Mit der Anordnung von Vliesmaterial oder jedem anderen saugfähigen Material in dem Zwischenraum ist ein wirksames und effektives Speichermedium für Flüssigkeit geschaffen, so dass die aus dem Innenschlot abgeführte Flüssigkeit zurückgehalten wird.

Vorteilhafterweise ist der Innenschlot bzw. die Wandung des Innenschlotes durch den Hohlkörper selbst und der Außenschlot bzw. die Wandung des Außenschlotes durch einen mit dem Hohlkörper verbindbaren, rohrförmigen Abschnitt des Mundstückes gebildet. Mundstück kann einstückig mit dem Hohlkörper ausgebildet sein. In anderen Ausführungsformen ist das Mundstück separat zum Hohlkörper ausgebildet und z.B. durch eine Steckverbindung, eine Schnapp- oder Schnellverschlusssystem mit dem Hohlkörper verbindbar. In montiertem Zustand, der dem Funktionszustand entspricht, bildet eine umlaufende Zwischenwand des Mundstückes die Wandung zum Vorratstank. Die Außenseite der Zwischenwandung ist mit einem Befestigungsmittel, z.B. einer Muffe, in Kontakt oder in anderen Ausführungen direkt mit der Flüssigkeit im Vorratstank. Die Innenseite der Zwischenwandung ist dem Zwischenraum zwischen Außenschlot und Innenschlot zugewandt. An der Innenseite der Zwischenwandung liegt das Vliesmaterial oder dergleichen an. Andere Ausbildungen und Anordnungen von Mundstück und/oder Hohlkörper sind aber ebenfalls möglich. Das Vliesmaterial oder dergleichen liegt somit sandwichartig zwischen der Wandung des Außenschlotes, durch welches Bauteil dieser auch immer gebildet ist, und der Wandung des Innenschlotes. Damit ist eine besonders kompakte Verdampferkartusche mit einer effektiven Absorptionsfähigkeit geschaffen.

In einer weiteren vorteilhaften Ausführungsform ist das Absorptionselement direkt auf der Innenseite des Schlotes angeordnet. In dieser Ausführungsform ist eine einwandige Ausbildung vorgesehen, bei der z.B. ein Vliesmaterial mit hoher Eigensteifigkeit als Absorptionselement vorgesehen ist. Der Strömungskanal ist somit direkt vom Vliesmaterial oder dergleichen umgeben, so dass sich das Kondensat direkt und unmittelbar auf dem Absorptionselement absetzt und von diesem aufgenommen wird, was eine effektive Abführung der Flüssigkeit aus dem Strömungskanal gewährleistet.

Vorzugsweise ist das Absorptionselement hülsenförmig ausgebildet und liegt an der Innenwand des Schlotes an. Damit ist das Absorptionselement einfach und platzsparend für die Bildung einer kompakten Verdampferkartusche montierbar.

Die Aufgabe wird auch durch einen Inhalator der eingangs gennannten Art dadurch gelöst, dass die Verdampferkartusche nach einem oder mehreren der Ansprüche 1 bis 9 ausgebildet und eingerichtet ist.

Die sich daraus ergebenden Vorteile wurden bereits im Zusammenhang mit der Verdampferkartusche beschrieben, weshalb zur Vermeidung von Wiederholungen auf die vorstehenden Ausführungen verwiesen wird.

Weitere zweckmäßige und/oder vorteilhafte Merkmale und Weiterbildungen zur Verdampferkartusche und dem Inhalator ergeben sich aus den Unteransprüchen und der Beschreibung. Besonders bevorzugte Ausführungsformen der Verdampferkartusche und des Inhalators werden anhand der beigefügten Zeichnung näher erläutert. In der Zeichnung zeigt:
- Fig. 1: eine schematische Darstellung einer ersten Ausführungsform eines erfindungsgemäßen Inhalators mit Kartuschenträger und Verdampferkartusche im Teilschnitt,
- Fig. 2: eine besonders bevorzugte Ausführungsform einer erfindungsgemäßen Verdampferkartusche im Schnitt, die mit einem angepassten Kartuschenträger zur Bildung eines erfindungsgemäßen Inhalators kombinierbar ist, und
- Fig. 3: eine schematische Darstellung einer weiteren Ausführungsform eines Inhalators mit Kartuschenträger und Verdampferkartusche im Teilschnitt.

Die in der Zeichnung dargestellte Verdampferkartusche sowie der Inhalator dienen zum Inhalieren von mit Wirkstoffen, z.B. Nikotin, angereicherten Aerosolen bzw. Aerosol-Dampf-Gemischen aus Flüssigkeiten, sind entsprechend im Zusammenhang mit einer E-Zigarette beschrieben. Die Verdampferkartusche und der Inhalator sind in gleicher Weise zum Inhalieren von mit medizinischen Wirkstoffen angereichertem Aerosol bzw. Aerosol-Dampfgemisch aus pharmazeutischen und/oder nahrungsergänzenden Produkten einsetzbar.

Die dargestellte Verdampferkartusche 10, insbesondere als Bestandteil eines Inhalators 11, umfasst einen Hohlkörper 12 mit einem durchgängigen, einen Strömungskanal 13 bildenden, barrierefreien Schlot 14 sowie einen Vorratstank 15 zum Bevorraten von Flüssigkeit 16, wobei der Vorratstank 15 mindestens eine Zugangsöffnung 17 zum Schlot 14 aufweist und im Bereich jeder Zugangsöffnung 17 eine sich über die gesamte Zugangsöffnung 17 erstreckende Verdampfereinheit 18 angeordnet ist, die ein Dochtorgan 19 und ein Heizorgan 20 zum Bilden von Aerosolen (bzw. Aerosol-Dampfgemischen) aus der aus dem Vorratstank 15 zugeführten Flüssigkeit 16 aufweist, wobei die Verdampfereinheit 18 dazu flüssigkeitspermeabel ausgebildet ist, derart, dass Flüssigkeit 16 mindestens initial kapillar aus dem Vorratstank 15 durch die Verdampfereinheit 18 in Richtung des Schlotes 14 und als Aerosol (bzw. Aerosol-Dampf-gemisch) innerhalb des Schlotes 14 in Richtung eines Mundstücks 21 förderbar ist. Der Einfachheit halber umfasst der Begriff Aerosol im Zusammenhang mit der Erfindung durchgehend auch Aerosol-Dampfgemische. Auch wenn von Dampf oder Gas die Rede ist, sind stets auch Aerosole und Aerosol-Dampf-Gemische gemeint.

Der Hohlkörper 12 mit seinem Schlot 14 bildet mindestens einen Strömungskanal 13. Die Form des Hohlkörpers 12 kann ebenso wie der Verlauf jedes Strömungskanals 13 nahezu beliebig sein. Entscheidend ist, dass die Eintrittsseite E_{S} jedes Strömungskanals 13 offen zur Umgebung ist, um z.B. Luft ansaugen zu können, und dass die Austrittsseite A_{S} offen ist, um ein z.B. einen Unterdruck anlegen zu können, insbesondere durch das Saugen einer konsumierenden Person. Offen bedeutet in diesem Zusammenhang, dass die Eintrittsseite E_{S} und die Austrittsseite A_{S} luftdurchlässig sind. Es können auch zwei oder mehr Strömungskanäle 13 mit entsprechenden Eintrittsseiten E_{S} und Austrittsseiten A_{S} vorgesehen sein. Mindestens von der Verdampfereinheit 18 bis zum Mundstück 21 ist jeder Strömungskanal 13 nicht nur luftdurchlässig, sondern vollständig frei von Hindernissen. Für den Fall, dass zwei oder mehr Strömungskanäle 13 vorgesehen sind, ist wahlweise jedem Strömungskanal 13 ein Absorptionselement 22 zugeordnet, oder es ist ein gemeinsames Absorptionselement 22 vorgesehen, das im Bereich eines gemeinsamen Abschnitts, vorzugsweis unmittelbar vor dem Austrittsseite A_{S}, des Schlotes 14 angeordnet ist.

Im Bereich der Zugangsöffnung 17 zwischen Vorratstank 15 und Strömungskanal 13 bildet die Verdampfereinheit 18 eine Art Flüssigkeitssperre, die verhindert, dass Flüssigkeit 16 aus dem Vorratstank 15 direkt und als Flüssigkeit 16 in den Strömungskanal 13 fließt. Unabhängig von der Form und Ausbildung des Vorratstanks 15, es können auch zwei oder mehr Vorratstanks 15 vorgesehen sein, und des Hohlkörpers 12 sowie der Anordnung/Positionierung von Vorratstank 15 zu Hohlkörper 12 stellt die Verdampfereinheit 18 sicher, dass Flüssigkeit 16 zwangsläufig aus dem Vorratstank 15 in Richtung des Strömungskanals 13 geführt wird und spätestens beim Austritt aus der Verdampfereinheit 18 als Dampf bzw. Aerosol bzw. Aerosol-Dampfgemisch in den oder jeden Strömungskanal 13 abgegeben wird. Das Mundstück 21 kann eine separate Komponente oder Bestandteil der Verdampferkartusche 10 sein.

Diese Verdampferkartusche 10 zeichnet sich dadurch aus, dass im Bereich des Schlotes 14 mindestens abschnittsweise mindestens ein Absorptionselement 22 angeordnet ist, das zum Aufnehmen von innerhalb des Schlotes 14 durch Kondensation des Aerosols (bzw. Aerosol-Dampfgemisches) gebildeten Flüssigkeitstropfen ausgebildet und eingerichtet ist. Es besteht demnach die Möglichkeit, dass dem Schlot 14 über seine gesamte Länge oder über Teile der Länge ein Absorptionselement 22 oder mehrere Absorptionselemente 22 zugeordnet sind bzw. ausgebildet sind.

Die Verdampferkartusche 10 kann als Einwegartikel eine bauliche Einheit sein, die die Komponenten Hohlkörper 12, Vorratstank 15 und Verdampfereinheit 18 beinhaltet. Die Verdampferkartusche 10 kann aber auch mehrteilig ausgebildet sein, wobei sich Komponenten der Verdampferkartusche 10 auf den Einwegartikel und den Mehrwegartikel verteilen, derart, dass z.B. der Vorratstank 15 ein Einwegartikel ist, der erst beim Zusammenführen mit einem Kartuschenträger 24, der ein Mehrwegartikel sein kann und neben einer elektronischen Steuereinheit 25 und einer Energiequelle 26 auch Bestandteile der Verdampferkartusche 10, wie z.B. den Hohlkörper 12 und die Verdampfereinheit 18, umfassen kann, zur baulichen Einheit der Verdampferkartusche 10 führt. Die Verdampferkartusche 10 definiert sich entsprechend über ihre Komponenten, nämlich Hohlkörper 12 mit den Strömungskanal 13 bildendem Schlot 14, Vorratstank 15 und Verdampfereinheit 18, und nicht über die konstruktive/bauliche Zuordnung der Komponenten zum Mehrwegartikel bzw. Einwegartikel.

Die im Folgenden beschriebenen Merkmale und Weiterbildungen stellen für sich betrachtet oder in Kombination miteinander bevorzugte Ausführungsformen dar. Es wird ausdrücklich darauf hingewiesen, dass Merkmale, die in den Ansprüchen und/oder der Beschreibung und/oder der Zeichnung zusammengefasst oder in einer gemeinsamen Ausführungsform beschrieben sind, auch funktional eigenständig die weiter oben beschriebene Verdampferkartusche 10 weiterbilden können.

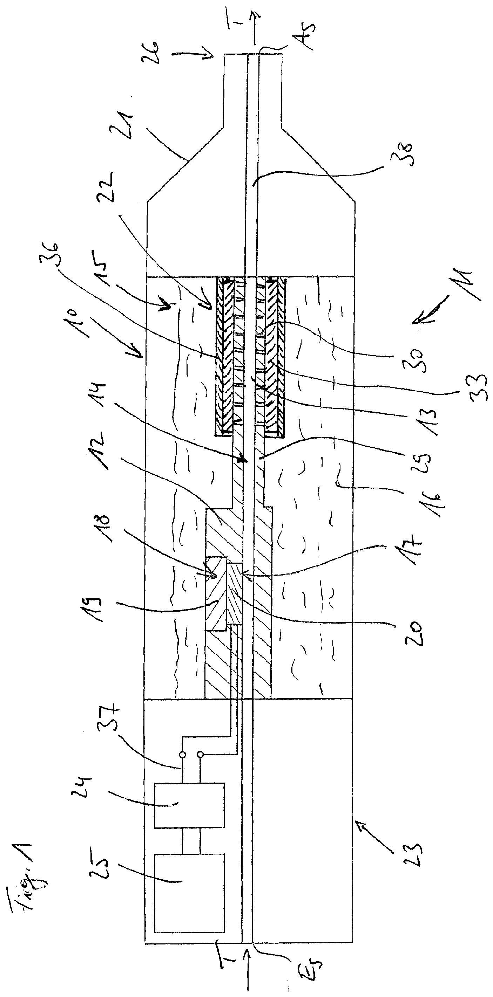

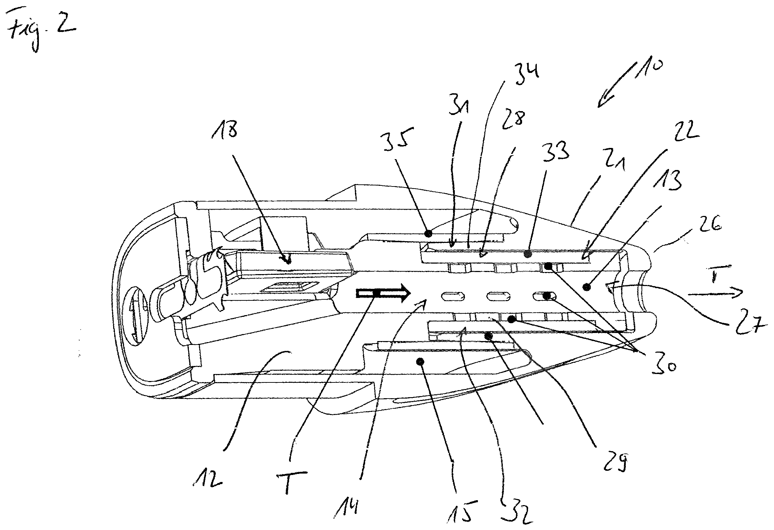

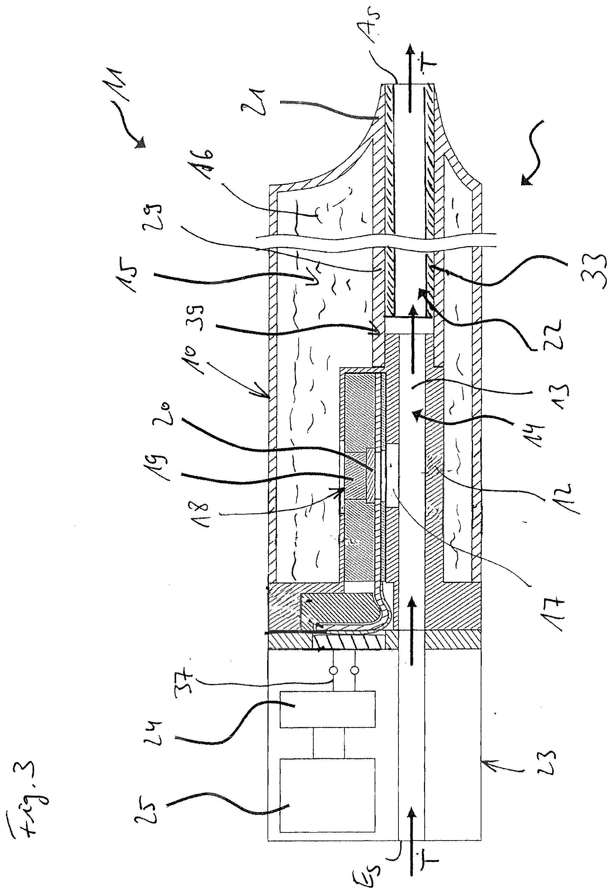

In den Figuren 1 bis 3 sind unterschiedliche Ausführungsformen der Verdampferkartusche 10 bzw. des entsprechenden Inhalators 11 gezeigt. Das oder jedes Absorptionselement 22 ist entlang des sich von der Verdampfereinheit 18 bis zum freien Ende 26 des Mundstückes 21 erstreckenden Abschnitts des Schlotes 14 in Strömungsrichtung T des Aerosols hinter der Verdampfereinheit 18 angeordnet. Das oder jedes Absorptionselement 22 kann einteilig oder mehrteilig ausgebildet sein. In anderen Ausführungsformen kann das Absorptionselement 22 auch integraler Bestandteil bestehender Komponenten der Verdampferkartusche 10, insbesondere des Hohlkörpers 12, sein. In der Ausführungsform gemäß Figur 1 ist ein einzelnes Absorptionselement 22 vorgesehen, das sich entlang eines Abschnitts des Schlots 14 erstreckt und zwischen der Verdampfereinheit 18 und dem Mundstück 21 positioniert ist. Das Absorptionselement 22 kann auch vollständig im Mundstück 21 angeordnet sein oder sich über die gesamte Länge von der Verdampfereinheit 18 bis zum freien Ende 26 des Mundstücks 21 erstrecken. Entlang dieser Strecke können auch mehrere Absorptionselemente 22 angeordnet sein. In der Figur 3 ist eine weitere Ausführungsform mit einem separat zum Hohlkörper 12 und/oder Schlot 14 ausgebildeten Absorptionselement 22 dargestellt. Auf die konkrete Ausbildung der Absorptionselemente 22 in den Ausführungsformen gemäß der Figuren 1 und 3 wird weiter unten eingegangen. Vorzugsweise wird das Absorptionselement 22 - mindestens teilweise bzw. abschnittsweise - durch den Schlot 14 selbst gebildet, der zur Bildung eines Innenschlotes 27 und eines Außenschlotes 28 mindestens abschnittsweise doppelwandig ausgebildet ist. In konstruktiv einfachen Ausführungsformen ist der durch den Hohlkörper 12 ausgebildeten Schlot 14 von Anfang bis Ende, also von der Eintrittsseite E_{S} bis zur Austrittsseite A_{S} doppelwandig ausgebildet. Der doppelwandige Schlot 14 kann aber auch durch mehrere separate Komponenten gebildet sein. Eine besonders bevorzugte Ausführungsform ist in der Figur 2 dargestellt. In dieser Ausführungsform sind der Innenschlot 27 und der Außenschlot 28 durch die Ausbildung von Hohlkörper 12 und Mundstück 21 einerseits und deren Positionierung zueinander andererseits gebildet. Der Außenschlot 28 grenzt direkt oder indirekt an den Vorratstank 15 und ist entsprechend dicht ausgebildet. Der Innenschlot 27 ist zur Herstellung einer Verbindung aus dem Strömungskanal 13 in den Außenschlot 28 mindestens abschnittsweise perforiert ausgebildet. Der Innenschlot 27 ist durch eine Bohrung/einen Durchgang im Hohlkörper 12 ausgebildet. Der Innenschlot 27 umgibt mit einer Wandung 29 somit den Strömungskanal 13. In der Wandung 29 des Innenschlotes 27 ist eine Perforation 30 ausgebildet, die aus feinsten Kapillaren, Bohrungen oder dergleichen gebildet sein kann. Der Außenschlot 28 ist durch eine umlaufende und gegenüber dem Vorratstank 15 vollständig geschlossene und flüssigkeitsdichte Wandung 31 des Mundstücks 21 gebildet. Andere Komponenten der Verdampferkartusche 10 können aber ebenfalls, ggf. ergänzt um separate Komponenten, zur Bildung von Innenschlot 27 und Außenschlot 28 eingesetzt werden.

In der dargestellten Ausführungsform sind die Wandung 29 des Innenschlotes 27 und die Wandung 31 des Außenschlotes 28 zur Bildung eines Zwischenraums 32 beabstandet zueinander angeordnet. Der Zwischenraum 32 kann z.B. zur Bildung eines isolierend wirkenden Luftpolsters und als Speicherraum für die dem Zwischenraum 32 zugeführte Flüssigkeit 26 frei/leer ausgebildet sein. In der dargestellten Ausführungsform ist im Zwischenraum 32 ein zusätzliches Absorptionsmittel 33, z.B. in Form eines Vliesmaterials, angeordnet. Das oder jedes Absorptionsmittel 33 kann den Zwischenraum 32 ganz oder teilweise ausfüllen. Neben Vliesmatten, Vliesstreifen oder dergleichen können auch anderen zur Absorption von Flüssigkeiten 26 geeignete Materialen eingesetzt und im Zwischenraum 32 angeordnet sein.

Wie bereits beschrieben, ist der Innenschlot 27 bzw. die Wandung 29 des Innenschlotes 27 durch den Hohlkörper 12 selbst und der Außenschlot 28 bzw. die Wandung 31 des Außenschlotes 28 durch das mit dem Hohlkörper 12 verbindbare Mundstück 21 gebildet. Dazu weist das Mundstück 21 zur Bildung der Wandung 31 einen rohrförmigen Abschnitt 34 auf. Zur Befestigung und Positionierung des Mundstücks 21 mit und am Hohlkörper 12 ist ein Befestigungsmittel 35, z.B. in Form einer Muffe, vorgesehen, mittels dem der rohrförmige Abschnitt 34 und damit das Mundstück 21 fixierbar ist. Selbstverständlich können Mundstück 21 und Hohlkörper 12 auch auf andere Weise miteinander verbunden sein. Des Weiteren kann auch die Ausbildung des Außenschlotes 28 konstruktiv anders realisiert sein.

In anderen Ausführungsformen, bei denen das oder jedes Absorptionselement 22 nicht durch den Schlot 14 selbst gebildet wird, ist diesem das oder jedes Absorptionselement 22 von innen und/oder außen zugeordnet. Bei der Ausführungsform gemäß Figur 1 umfasst das Absorptionselement 22 einen hülsenförmigen Rohrabschnitt 36 sowie ein zusätzliches Absorptionsmittel 33. Das Absorptionselement 22 liegt außen am Schlot 14 an. Das Mundstück 21 ist separat zur Verdampferkartusche 10 ausgebildet, also gegenüber dem Hohlkörper 12 eine eigenständige Komponente. Im Mundstück 21 ist eine Bohrung 38 oder dergleichen zur Verlängerung des Schlotes 14 ausgebildet, wobei das Absorptionselement 22 am Ende des im Hohlkörper 12 gebildeten Schlotes 14 endet. Zum Abführen von kondensierten Aerosolen bzw. Aerosol-Dampf-Gemischen, also Flüssigkeitstropfen an der Wandung 29 des Schlotes 14, ist die Wandung 29 des Schlotes 14 in dem Bereich, in dem das Absorptionselement 22 angeordnet ist, mit der Perforation 30 versehen. Der Rohrabschnitt 36 schirmt das zusätzliche Absorptionsmittel 33, das zwischen dem Rohrabschnitt 36 und dem Hohlkörper 12 angeordnet ist, vollständig und dichtend gegenüber dem Vorratstank 15 ab. Außerhalb der Ummantelung durch das Rohrelement 36 ist der durch den Hohlkörper 12 gebildete Schlot 14 ebenfalls ohne Perforation 30, also dicht gegenüber dem Vorratstank 15, ausgebildet.

Die Figur 3 zeigt eine Ausführungsform, bei der das Absorptionselement 22 das Absorptionsmittel 33 ist. Das Absorptionselement 22 bzw. das Absorptionsmittel 33 ist direkt auf der Innenseite des Schlotes 14 angeordnet wobei der Schlot 14 durch den Hohlkörper 12 und einen rohrförmigen Abschnitt 39 des Mundstücks 21 gebildet ist. Das Absorptionsmittel 33 kann z.B. aus Vliesmaterial gebildet sein. Bevorzugt ist das Absorptionselement 22 bzw. das Absorptionsmittel 33 hülsenförmig ausgebildet und liegt innen an der Wandung 29 des Schlotes 14 an. Damit das Absorptionsmittel 33 aus Vliesmaterial im Inneren des Schlotes 14 hält und ausreichend fixiert ist, weist das Vliesmaterial oder jedes andere geeignete Absorptionsmedium eine ausreichende Eigensteifigkeit auf.

Bevorzugt ist die Verdampferkartusche 10 zum mechanischen und elektrischen Verbinden mit dem mindestens die elektronische Steuereinheit 24 und die Energiequelle 25 umfassenden Kartuschenträger 23 zur Bildung des Inhalators 11 ausgebildet und eingerichtet, wobei die Verdampfereinheit 19 elektrische Kontakte 37 zur elektrischen Kontaktierung mit der Energiequelle 25 umfasst. Der Inhalator 11 kann z.B. durch eine inhalierende Person aktiviert werden, beispielsweise als E-Zigarette, oder z.B. durch eine Pumpe aktiviert werden, z.B. als medizinisches Instrument für den Fall, das die Person selbst nicht mehr oder nicht ausreichend saugen kann.

Das Funktionsprinzip des erfindungsgemäßen Inhalators 11 wird beispielhaft anhand einer E-Zigarette als Inhalator 11 insbesondere mit Bezug auf die Figur 1 beschrieben. Der Inhalator 11 ist zum Inhalieren von mit Wirkstoffen angereichertem Aerosol bzw. Aerosol-Dampf-Gemisch ausgebildet und eingerichtet und umfasst einen mindestens den die elektronische Steuereinheit 24 und die Energiequelle 25 umfassenden Kartuschenträger 23 sowie eine Verdampferkartusche 10, die sich erfindungsgemäß dadurch auszeichnet, dass sie nach einem oder mehreren der Ansprüche 1 bis 6 ausgebildet und eingerichtet ist.

Eine konsumierende Person saugt z.B. an dem Mundstück 21 des Inhalators 11, der aus dem Kartuschenträger 23 und der Verdampferkartusche 10 gebildet ist, wobei sich in dem Vorratstank 15 der Verdampferkartusche 10 eine Flüssigkeit 26 befindet, die beispielsweise Glycerin, Propylenglycol und ggf. weitere Wirkstoffe und/oder Geschmacksstoffe enthält. Durch das Saugen wird in dem Strömungskanal 13 ein Unterdruck erzeugt, der seinerseits z.B. über einen nicht dargestellten Sensor die Steuereinheit 24 aktiviert. Die Steuereinheit 24 steuert das Heizorgan 20, das von der Energiequelle 25 mit Energie versorgt wird. Flüssigkeit 26 aus dem Vorratstank 15 wird mittels des Dochtorgans 19 mindestens initial kapillar durch Mikrokanäle aus dem Vorratstank 15 in Richtung des Heizorganes 20 transportiert. Am bzw. im erwärmten Heizorgan 20 wird die Flüssigkeit 26 zu Dampf umgewandelt, wobei das Heizorgan 21 die Flüssigkeit 26 bzw. den daraus gebildeten Dampf aufgrund der flüssigkeits- und dampfpermeablen Struktur in Richtung des Strömungskanals 13 transportiert und an diesen abgibt. Beim Eintritt des Dampfes in den Strömungskanal 13 entstehen durch den Luftstrom Aerosole bzw. ein Aerosol-Dampf-Gemisch, das von der konsumierenden Person angesaugt und inhaliert wird. Sich möglicherweise auf dem Weg von der Verdampfereinheit 18 bis zum Mundstück 21 insbesondere durch Kondensation bildende Flüssigkeitstropfen werden von dem oder jedem Absorptionselement 22 aus dem Innenschlot 27 abgeführt, aufgenommen und ggf. gespeichert.

## Patentansprüche

1. Verdampferkartusche (10) als Bestandteil eines Inhalators (11), umfassend einen Hohlkörper (12) mit einem durchgängigen, einen Strömungskanal (13) bildenden, barrierefreien Schlot (14) sowie einen Vorratstank (15) zum Bevorraten von Flüssigkeit (16), wobei der Vorratstank (15) mindestens eine Zugangsöffnung (17) zum Schlot (14) aufweist und im Bereich jeder Zugangsöffnung (17) eine sich über die gesamte Zugangsöffnung (17) erstreckende Verdampfereinheit (18) angeordnet ist, die ein Dochtorgan (19) und ein Heizorgan (20) zum Bilden von Aerosolen aus der aus dem Vorratstank (15) zugeführten Flüssigkeit (16) aufweist, wobei die Verdampfereinheit (18) dazu flüssigkeitspermeabel ausgebildet ist, derart, dass Flüssigkeit (16) mindestens initial kapillar aus dem Vorratstank (15) durch die Verdampfereinheit (18) in Richtung des Schlotes (14) und als Aerosol innerhalb des Schlotes (14) in Richtung eines Mundstücks (21) förderbar ist, wobei im Bereich des Schlotes (14) mindestens abschnittsweise mindestens ein Absorptionselement (22) angeordnet ist, das zum Aufnehmen von innerhalb des Schlotes (14) durch Kondensation des Aerosols gebildeten Flüssigkeitstropfen ausgebildet und eingerichtet ist, wobei das Absorptionselement (22) durch den Schlot (14) selbst gebildet wird, **dadurch gekennzeichnet, dass** der Schlot (14) zur Bildung eines Innenschlots (27) und eines Außenschlots (28) mindestens abschnittsweise doppelwandig ausgebildet ist, wobei der Außenschlot (28) an den Vorratstank (15) grenzt und entsprechend dicht ausgebildet ist, während der Innenschlot (27) zur Herstellung einer Verbindung aus dem Strömungskanal (13) in den Außenschlot (28) mindestens abschnittsweise perforiert ausgebildet ist, wobei eine Wandung (29) des Innenschlotes (27) und eine Wandung (31) des Außenschlotes (28) zur Bildung eines Zwischenraums (32) beabstandet zueinander angeordnet sind.

2. Verdampferkartusche (10) nach Anspruch 1, **dadurch gekennzeichnet, dass** das oder jedes Absorptionselement (22) entlang des sich von der Verdampfereinheit (18) bis zum freien Ende (26) des Mundstückes (21) erstreckenden Abschnitts des Schlotes (14) in Strömungsrichtung (T) des Aerosols hinter der Verdampfereinheit (18) angeordnet ist.

3. Verdampferkartusche (10) nach Anspruch 1 oder 2, **dadurch gekennzeichnet, dass** im Zwischenraum (32) ein zusätzliches Absorptionsmittel (33), z.B. in Form eines Vliesmaterials, angeordnet ist.

4. Verdampferkartusche (10) nach einem oder mehreren der Ansprüche 1 bis 3, **dadurch gekennzeichnet, dass** der Innenschlot (27) bzw. die Wandung (29) des Innenschlotes (27) durch den Hohlkörper (12) selbst und der Außenschlot (28) bzw. die Wandung (31) des Außenschlotes (28) durch einen mit dem Hohlkörper (12) verbindbaren, rohrförmigen Abschnitt (34) des Mundstückes (21) gebildet ist.

5. Verdampferkartusche (10) nach Anspruch 1 oder 2, **dadurch gekennzeichnet, dass** das Absorptionselement (22) direkt auf der Innenseite des Schlotes (14) angeordnet ist.

6. Verdampferkartusche (10) nach Anspruch 5, **dadurch gekennzeichnet, dass** das Absorptionselement (22) hülsenförmig ausgebildet ist und an der Innenwand des Schlotes (14) anliegt.

7. Inhalator (11), ausgebildet und eingerichtet zum Inhalieren von mit Wirkstoffen angereichertem Aerosol, umfassend einen mindestens eine elektronische Steuereinheit (24) und eine Energiequelle (25) umfassenden Kartuschenträger (23) sowie eine Verdampferkartusche (10), **dadurch gekennzeichnet, dass** die Verdampferkartusche (10) nach einem oder mehreren der Ansprüche 1 bis 6 ausgebildet und eingerichtet ist.

## Claims

1. Vaporizer cartridge (10) as part of an inhaler (11), comprising a housing (12) having a continuous, barrier-free vent (14) forming a flow channel (13), and a storage tank (15) for the storage of liquid (16), wherein the storage tank (15) has at least one access opening (17) to the vent (14) and in the region of each access opening (17) there is arranged a vaporizer unit (18) which extends over the entire access opening (17), said vaporizer unit having a wick member (19) and a heating member (20) for the formation of aerosols from the liquid (16) supplied from the storage tank (15), wherein the vaporizer unit (18) is for that purpose configured so as to be liquid-permeable, such that liquid (16) can be conveyed, at least initially in a capillary manner, from the storage tank (15) through the vaporizer unit (18) in the direction of the vent (14) and, in the form of an aerosol, inside the vent (14) in the direction of a mouthpiece (21), **wherein** there is arranged in the region of the vent (14), at least in some portions, at least one absorption element (22) which is configured and adapted for the absorption of liquid drops formed inside the vent (14) by condensation of the aerosol, wherein the absorption element (22) is formed by the vent (14) itself, **characterized in that** said vent being of double-walled form at least in some portions to form an inner vent (27) and an outer vent (28), wherein the outer vent (28) adjoins the storage tank (15) and is accordingly configured so as to be impermeable, while the inner vent (27) is configured so as to be perforated at least in some portions in order to produce a connection from the flow channel (13) into the outer vent (28), wherein a wall (29) of the inner vent (27) and a wall (31) of the outer vent (28) are arranged spaced apart from one another to form an intermediate space (32).

2. Vaporizer cartridge (10) according to claim 1, **characterized in that** the or each absorption element (22) is arranged along the portion of the vent (14) that extends from the vaporizer unit (18) to the free end (26) of the mouthpiece (21), behind the vaporizer unit (18) in the flow direction T of the aerosol.

3. Vaporizer cartridge (10) according to claim 1 or 2, **characterized in that** an additional absorption means (33), for example in the form of a bonded fabric material, is arranged in the intermediate space (32).

4. Vaporizer cartridge (10) according to one or more of claims 1 to 3, **characterized in that** the inner vent (27), or the wall (29) of the inner vent (27), is formed by the housing (12) itself, and the outer vent (28), or the wall (31) of the outer vent (28), is formed by a tubular portion (34) of the mouthpiece (21) which can be connected to the housing (12).

5. Vaporizer cartridge (10) according to claim 1 or 2, **characterized in that** the absorption element (22) is arranged directly on the inner side of the vent (14).

6. Vaporizer cartridge (10) according to claim 5, **characterized in that** the absorption element (22) is of sleeve-like form and lies against the inner wall of the vent (14).

7. Inhaler (11), configured and adapted for the inhalation of aerosol enriched with active substances, comprising a cartridge carrier (23) comprising at least an electronic control unit (24) and a power source (25), and a vaporizer cartridge (10), **characterized in that** the vaporizer cartridge (10) is configured and adapted according to one or more of claims 1 to 6.

## Revendications

1. Cartouche d'évaporation (10) en tant que constituant d'un inhalateur (11), comprenant un logement (12) avec un conduit continu (14) sans barrière formant un canal d'écoulement (13) ainsi qu'un réservoir de stockage (15) pour stocker du liquide (16), le réservoir de stockage (15) présentant au moins une ouverture d'accès (17) vers le conduit (14) et une unité d'évaporation (18) s'étendant sur toute l'ouverture d'accès (17) étant agencée dans la zone de chaque ouverture d'accès (17), laquelle unité d'évaporation présente un organe écouvillon (19) et un organe chauffant (20) pour former des aérosols à partir du liquide (16) acheminé depuis le réservoir de stockage (15), l'unité d'évaporation (18) étant configurée pour être perméable aux liquides, de telle sorte que le liquide (16) puisse être transporté au moins initialement par capillarité depuis le réservoir de stockage (15) à travers l'unité d'évaporation (18) en direction du conduit (14) et sous forme d'aérosol à l'intérieur du conduit (14) en direction d'une embouchure (21), au moins un élément d'absorption (22) étant agencé au moins par sections dans la zone du conduit (14), qui est configuré et adapté pour recevoir les gouttes de liquide formées à l'intérieur du conduit (14) par condensation de l'aérosol, l'élément d'absorption (22) étant formé par le conduit (14) lui-même, **caractérisée en ce que** le conduit (14) est configuré à double paroi au moins par sections afin de former un conduit intérieur (27) et un conduit extérieur (28), le conduit extérieur (28) étant adjacent au réservoir de stockage (15) et étant configuré de manière étanche en conséquence, tandis que le conduit intérieur (27) est configuré sous forme perforée au moins par sections pour établir une connexion depuis le canal d'écoulement (13) vers le conduit extérieur (28), une paroi (29) du conduit intérieur (27) et une paroi (31) du conduit extérieur (28) étant agencées à distance l'une de l'autre pour former un espace intermédiaire (32).

2. Cartouche d'évaporation (10) selon la revendication 1, **caractérisée en ce que** le ou chaque élément d'absorption (22) est agencé derrière l'unité d'évaporation (18) dans la direction d'écoulement (T) de l'aérosol le long de la section du conduit (14) s'étendant de l'unité d'évaporation (18) jusqu'à l'extrémité libre (26) de l'embouchure (21).

3. Cartouche d'évaporation (10) selon la revendication 1 ou 2, **caractérisée en ce qu'**un agent d'absorption supplémentaire (33), par exemple sous la forme d'un matériau nappé, est agencé dans l'espace intermédiaire (32).

4. Cartouche d'évaporation (10) selon une ou plusieurs des revendications 1 à 3, **caractérisée en ce que** le conduit intérieur (27) ou la paroi (29) du conduit intérieur (27) est formé par le logement (12) lui-même et le conduit extérieur (28) ou la paroi (31) du conduit extérieur (28) est formé par une section tubulaire (34) de l'embouchure (21) pouvant être reliée au logement (12).

5. Cartouche d'évaporation (10) selon la revendication 1 ou 2, **caractérisée en ce que** l'élément d'absorption (22) est agencé directement sur le côté intérieur du conduit (14).

6. Cartouche d'évaporation (10) selon la revendication 5, **caractérisée en ce que** l'élément d'absorption (22) est configuré en forme de douille et repose contre la paroi intérieure du conduit (14).

7. Inhalateur (11), configuré et adapté pour inhaler un aérosol enrichi en principes actifs, comprenant un support de cartouche (23) comprenant au moins un organe de commande électronique (24) et une source d'énergie (25), ainsi qu'une cartouche d'évaporation (10), **caractérisé en ce que** la cartouche d'évaporation (10) est configurée et adaptée selon une ou plusieurs des revendications 1 à 6.
